# EUROPEAN PATENT APPLICATION

(11) **EP 3 288 290 A1**
(43) Date of publication of application: **28.02.2018**
(21) Application number: 16782982.9
(22) Date of filing: 04.04.2016
(51) Int. Cl.: H04R 17/00, A61B 8/12, A61B 8/14, G01N 29/24, H04R 31/00

(54) **ULTRASONIC TRANSDUCER, ULTRASONIC PROBE, AND METHOD FOR MANUFACTURING ULTRASONIC TRANSDUCER**

(30) Priority: 21.04.2015 JP 2015086781
(71) Applicant: Olympus Corporation, Hachioji-shi, Tokyo 192-8507 (JP)
(72) Inventor: WAKABAYASHI, Katsuhiro, Hachioji-shi Tokyo 192-8507 (JP)
(74) Representative: Gunzelmann, Rainer
(86) International application number: PCT/JP2016/061046
(87) International publication number: WO 2016/170961

(57) **Abstract**

An ultrasound transducer according to the present invention includes: a plurality of piezoelectric elements; a substrate configured to perform input and output of an electric signal with respect to the piezoelectric elements; a plurality of signal input and output electrodes where each signal input and output electrode is provided between each piezoelectric element and the substrate and configured to electrically connect the piezoelectric element with the substrate; a plurality of backing materials where each backing material is provided at each of the piezoelectric elements on a side where the signal input and output electrode is arranged and configured to attenuate a force generated by operation of the piezoelectric element; and a plurality of sealing portions where each sealing portion is configured to seal an outer surface of at least a portion of an electrical path connecting the substrate with the signal input and output electrode. The plurality of piezoelectric elements, the plurality of backing materials, a portion of the substrate, the plurality of signal input and output electrodes, and the plurality of sealing portions are obtained by dividing a forming member along a stacking direction of the forming member, the forming member being formed by stacking a plurality of materials, each of the plurality of materials forming the piezoelectric elements, the backing materials, the portion of the substrate, the signal input and output electrodes, and the sealing portions.

## Description

### Field

The present invention relates to an ultrasound transducer configured to emit an ultrasound to an observation target, receive an ultrasound echo reflected on the observation target, convert the ultrasound echo into an echo signal, and output the echo signal, an ultrasound probe and a method of manufacturing an ultrasound transducer.

### Background

Ultrasound is applied in some cases for observing a characteristic of a living tissue or material as an observation target. Specifically, the ultrasound transducer transmits ultrasound to the observation target, receives an ultrasound echo reflected by the observation target, and an ultrasound observation apparatus performs predetermined signal processing on the received ultrasound echo, whereby information related to the characteristic of the observation target can be obtained.

The ultrasound transducer includes a plurality of piezoelectric elements that converts an electrical pulse signal into an ultrasound pulse (acoustic pulse), emits the ultrasound pulse to the observation target, converts an ultrasound echo reflected on the observation target into an electrical echo signal expressed by a voltage change, and outputs the echo signal (refer to Patent Literature 1, for example). The ultrasound echo is obtained from the observation target, for example, by arranging the plurality of piezoelectric elements in an array pattern, electronically switching the elements related to transmission and reception, or delaying transmission and reception of the piezoelectric body of each of the ultrasound transducer.

Meanwhile, each of the piezoelectric elements is electrically connected by wiring to a circuit board configured to transmit a pulse signal and receive an echo signal. The piezoelectric element and the wiring are connected by soldering, for example, and the heat at the time of soldering might induce degradation of the characteristics of the piezoelectric element, in the form of depolarization, or the like.

As a technique for suppressing depolarization of the piezoelectric element, there is a disclosed technique of forming a conductive thin film for providing electrical connection to the circuit board on a side surface of a base material forming the piezoelectric element and then dividing the base material after formation of the thin film so as to be able to electrically connect a plurality of piezoelectric elements with a circuit board without soldering (for example, refer to Patent Document 2).

### Citation List

### Patent Literature

Patent Literature 1: JP 2002-224104 A
Patent Literature 2: JP 2007-201901 A

### Summary

### Technical Problem

In recent years, for example, there is a demand for miniaturization of the ultrasound transducer, for a case where the ultrasound transducer is mounted on an endoscope to be inserted in a subject for examining the interior of the subject. In miniaturization of the ultrasound transducer, it is desirable to reduce the pitch of the plurality of piezoelectric elements. With the technique disclosed in Patent Literature 2, however, the piezoelectric thin film might be detached from the base material (piezoelectric element) in some cases due to stress generated at the time of dividing the base material at a narrow pitch.

The present invention has been made in view of the above-described issue, and it is an object of the present invention to provide an ultrasound transducer, an ultrasound probe, and an ultrasound transducer manufacturing method capable of suppressing degradation of characteristics of a piezoelectric element and realizing a narrow pitch in the plurality of piezoelectric elements. Solution to Problem

To solve the problem described above and to achieve the object, an ultrasound transducer according to the present invention includes: a plurality of piezoelectric elements configured to emit ultrasound according to an input of an electric signal and to convert ultrasound incident from outside into an electric signal; a substrate configured to perform input and output of an electric signal with respect to each of the piezoelectric elements; a plurality of signal input and output electrodes where each signal input and output electrode is provided between each piezoelectric element and the substrate and is configured to electrically connect the piezoelectric element with the substrate; a plurality of backing materials where each backing material is provided at each of the piezoelectric elements on a side where the signal input and output electrode is arranged and is configured to attenuate ultrasound vibration generated by operation of the piezoelectric element; and a plurality of sealing portions where each sealing portion is configured to seal an outer surface of at least a portion of an electrical path connecting the substrate with the signal input and output electrode. The plurality of piezoelectric elements, the plurality of backing materials, a portion of the substrate, the plurality of signal input and output electrodes, and the plurality of sealing portions are obtained by dividing a forming member along a stacking direction of the forming member, the forming member being formed by stacking a plurality of materials, each of the plurality of materials forming the piezoelectric elements, the backing materials, the substrate, the signal input and output electrodes, and the sealing portions.

The ultrasound transducer according to the present invention further includes: a wall configured to surround a plurality of oscillating portions including at least the piezoelectric element, the backing material, the signal input and output electrode, and the sealing portion; and a second backing material provided in a hollow space formed by the wall and the plurality of oscillating portions, and configured to attenuate the ultrasound vibration.

In the ultrasound transducer according to the present invention, the signal input and output electrode includes a thick portion forming a portion of the electrical path.

The ultrasound transducer according to the present invention includes a connection electrode connected to each of the substrate and the signal input and output electrode, and configured to form the electrical path.

In the ultrasound transducer according to the present invention, a portion of the substrate is held by the backing material, and the connection electrode is connected to the substrate via a side surface of the backing material.

In the ultrasound transducer according to the present invention, the connection electrode includes a thin film formed by a physical vapor deposition method and a plating film formed by wet plating.

In the ultrasound transducer according to the present invention, a portion of the substrate is embedded in the backing material.

In the ultrasound transducer according to the present invention, a portion of the substrate is provided along a side surface of the backing material.

The ultrasound transducer according to the present invention further includes: a second electrode paired with the signal input and output electrode; and an acoustic matching layer provided at the piezoelectric element on a side opposite to a side on which the backing material is arranged and configured to adjust acoustic impedance of the ultrasound. The second electrode is grounded to a ground potential via conductive resin provided between the acoustic matching layer and the piezoelectric element.

In the ultrasound transducer according to the present invention, the plurality of piezoelectric elements is arranged in a scanning direction along which the forming member is divided and in an elevation direction substantially orthogonal to the scanning direction.

An ultrasound probe according to the present invention includes the ultrasound transducer according to the present invention at a distal end of the ultrasound probe.

A method of manufacturing an ultrasound transducer according to the present invention includes: a stacked member production process of stacking a plurality of materials to produce a stacked member, each of the materials forming: a plurality of piezoelectric elements configured to emit ultrasound according to an input of an electric signal and to convert ultrasound incident from outside into an electric signal; a portion of a substrate configured to perform input and output of an electric signal with respect to each of the piezoelectric elements; a plurality of signal input and output electrodes where each signal input and output electrode is provided between each piezoelectric element and the substrate and is configured to electrically connect the piezoelectric element with the substrate; and a plurality of backing materials where each backing material is provided at each of the piezoelectric elements on a side where the signal input and output electrode is arranged and is configured to attenuate ultrasound vibration generated by operation of the piezoelectric element; a forming member production process of sealing, with respect to the stacked member, an outer surface of at least a portion of an electrical path connecting the substrate with the signal input and output electrode to produce a forming member; and a forming process of dividing the forming member produced by the forming member production process along a stacking direction of the forming member to form the piezoelectric element, the backing material, a portion of the substrate, the signal input and output electrode, and the sealing portion.

In the method of manufacturing an ultrasound transducer according to the present invention, the stacked member production process includes a connection electrode member arrangement process of arranging a connection electrode member for forming a connection electrode connected to each of the substrate and the signal input and output electrode, the connection electrode being configured to form the electrical path.

In the method of manufacturing an ultrasound transducer according to the present invention, the connection electrode member arrangement process includes: a first process of forming a thin film on an outer surface corresponding to the electrical path by a physical vapor deposition method; and a second process of forming a plating film on an outer surface of the thin film by wet plating.

In the method of manufacturing an ultrasound transducer according to the present invention, the forming member is obtained by stacking a material forming the piezoelectric element, a material forming the signal input and output electrode, and a material forming the backing material in this order, and by causing the substrate to be held by the backing material. The forming process includes cutting the forming member such that a distance between adjacent members formed by division increases in a direction from the piezoelectric element toward the substrate.

### Advantageous Effects of Invention

According to the present invention, it is possible to suppress, in an ultrasound transducer, degradation of characteristics of the piezoelectric element in manufacture and to realize a narrow pitch in the plurality of piezoelectric elements.

### Brief Description of Drawings

FIG. 1 is a diagram schematically illustrating an endoscope system according to a first embodiment of the present invention.
FIG. 2 is a perspective view illustrating an ultrasound transducer according to the first embodiment of the present invention.
FIG. 3 is a schematic diagram illustrating a configuration of a main portion of the ultrasound transducer according to the first embodiment of the present invention.
FIG. 4 is a schematic diagram illustrating a configuration of a main portion of the ultrasound transducer according to the first embodiment of the present invention.
FIG. 5 is a schematic diagram illustrating a method of manufacturing the ultrasound transducer according to the first embodiment of the present invention.
FIG. 6 is a schematic diagram illustrating a method of manufacturing the ultrasound transducer according to the first embodiment of the present invention.
FIG. 7 is a schematic diagram illustrating a method of manufacturing the ultrasound transducer according to the first embodiment of the present invention.
FIG. 8 is a schematic diagram illustrating a method of manufacturing the ultrasound transducer according to the first embodiment of the present invention.
FIG. 9 is a schematic diagram illustrating a method of manufacturing the ultrasound transducer according to the first embodiment of the present invention.
FIG. 10 is a schematic diagram illustrating a method of manufacturing the ultrasound transducer according to the first embodiment of the present invention.
FIG. 11 is a schematic diagram illustrating a method of manufacturing the ultrasound transducer according to the first embodiment of the present invention.
FIG. 12 is a schematic diagram illustrating a method of manufacturing the ultrasound transducer according to the first embodiment of the present invention.
FIG. 13 is a schematic diagram illustrating a configuration of an ultrasound transducer according to a first modification of the first embodiment of the present invention.
FIG. 14 is a schematic diagram illustrating a configuration of an ultrasound transducer according to a second modification of the first embodiment of the present invention.
FIG. 15 is a schematic diagram illustrating a production of a forming member of the ultrasound transducer according to the second modification of the first embodiment of the present invention.
FIG. 16 is a schematic diagram illustrating a production of the forming member of the ultrasound transducer according to the second modification of the first embodiment of the present invention.
FIG. 17 is a schematic diagram illustrating a configuration of an ultrasound transducer according to a third modification of the first embodiment of the present invention.
FIG. 18 is a schematic diagram illustrating a production of a forming member of the ultrasound transducer according to the third modification of the first embodiment of the present invention.
FIG. 19 is a schematic diagram illustrating a configuration of a main portion of an ultrasound transducer according to a fourth modification of the first embodiment of the present invention.
FIG. 20 is a schematic diagram illustrating a configuration of a main portion of an ultrasound transducer according to a fifth modification of the first embodiment of the present invention.
FIG. 21 is a schematic diagram illustrating a configuration of an ultrasound transducer according to a sixth modification of the first embodiment of the present invention.
FIG. 22 is a schematic diagram illustrating a configuration of an ultrasound transducer according to a seventh modification of the first embodiment of the present invention.
FIG. 23 is a schematic diagram illustrating a configuration of an ultrasound transducer according to an eighth modification of the first embodiment of the present invention.
FIG. 24 is a schematic diagram illustrating a configuration of an ultrasound transducer according to a ninth modification of the first embodiment of the present invention.
FIG. 25 is a diagram illustrating a method of manufacturing an ultrasound transducer according to a tenth modification of the first embodiment of the present invention.
FIG. 26 is a schematic diagram illustrating a configuration of the ultrasound transducer according to the tenth modification of the first embodiment of the present invention.
FIG. 27 is a schematic diagram illustrating a configuration of an ultrasound transducer according to a second embodiment of the present invention.
FIG. 28 is a diagram illustrating a method of manufacturing the ultrasound transducer according to the second embodiment of the present invention.
FIG. 29 is a schematic diagram illustrating a method of manufacturing the ultrasound transducer according to the second embodiment of the present invention.
FIG. 30 is a schematic diagram illustrating a method of manufacturing the ultrasound transducer according to the second embodiment of the present invention.
FIG. 31 is a schematic diagram illustrating a method of manufacturing the ultrasound transducer according to the second embodiment of the present invention.
FIG. 32 is a schematic diagram illustrating a method of manufacturing the ultrasound transducer according to the second embodiment of the present invention.

### Description of Embodiments

Hereinafter, embodiments of the present invention (hereinafter, referred to as embodiment(s)) will be described with reference to the drawings. Note that the present invention is not limited by the following embodiments. In the drawings, same reference signs are attached to the same portions.

### (First Embodiment)

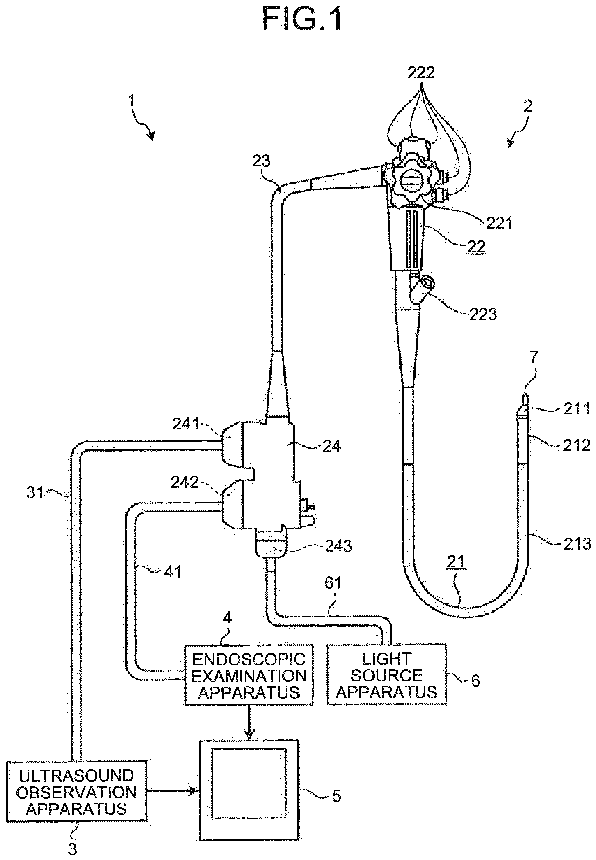

FIG. 1 is a diagram schematically illustrating an endoscope system 1 according to a first embodiment of the present invention. The endoscope system 1 is a system for performing ultrasound diagnosis of internal portions of a subject such as a human using an ultrasound endoscope. As illustrated in FIG. 1, the endoscope system 1 includes an ultrasound endoscope 2, an ultrasound observation apparatus 3, an endoscopic examination apparatus 4, a display device 5, and a light source apparatus 6.

The ultrasound endoscope 2, using its distal end portion, converts an electrical pulse signal received from the ultrasound observation apparatus 3 into an ultrasound pulse (acoustic pulse) and emits it to the subject, and also converts an ultrasound echo reflected on the subject into an electrical echo signal expressed by a voltage change and outputs the signal.

The ultrasound endoscope 2 typically includes imaging optics and imaging elements. The ultrasound endoscope 2 can be inserted into gastrointestinal tracts (esophagus, stomach, duodenum, and large intestine) or respiratory organs (trachea, bronchus) of the subject and can capture gastrointestinal tract, respiratory organs, and their surrounding organs (pancreas, gall bladder, bile duct, biliary tract, lymph nodes, mediastinal organs, blood vessels, or the like). The ultrasound endoscope 2 includes a light guide that guides illumination light emitted to the subject at the time of imaging. The light guide is configured such that a distal end portion thereof reaches a distal end of an insertion unit of the ultrasound endoscope 2 into the subject, while a proximal end portion thereof is connected to the light source apparatus 6 that generates illumination light.

As illustrated in FIG. 1, the ultrasound endoscope 2 includes an insertion unit 21, an operating unit 22, a universal cable 23, and a connector 24. The insertion unit 21 is a portion to be inserted into the subject. As illustrated in FIG. 1, the insertion unit 21 includes an ultrasound transducer 7, a rigid member 211, a bending portion 212, and a flexible tube portion 213. The ultrasound transducer 7 is provided at a distal end side of the insertion unit. The rigid member 211 is coupled to a proximal end side of the ultrasound transducer 7. The bending portion 212 is a bendable portion coupled to the proximal end side of the rigid member 211. The flexible tube portion 213 is a flexible portion coupled to the proximal end side of the bending portion 212. While specific illustration is omitted herein, the insertion unit 21 internally includes a light guide for transmitting illumination light supplied from the light source apparatus 6, a plurality of signal cables for transmitting various signals, and a processing instrument insertion passage for inserting treatment instruments.

The ultrasound transducer 7 may be any of a convex transducer, a linear transducer, and a radial transducer. The ultrasound endoscope 2 may be configured to cause the ultrasound transducer 7 to perform mechanical scan, or may provide, as the ultrasound transducer 7, a plurality of piezoelectric elements in an array pattern, and may cause the ultrasound transducer 7 to perform electronic scan by electronically switching the piezoelectric elements related to transmission and reception or by imposing delay onto transmission and reception of each of the piezoelectric elements. The configuration of the piezoelectric element will be described below.

The operating unit 22 is coupled to the proximal end side of the insertion unit 21 and receives various types of operation from a doctor, or the like. As illustrated in FIG. 1, the operating unit 22 includes a bending knob 221 for performing bending operation on the bending portion 212, and a plurality of operating members 222 for performing various types of operation. Moreover, the operating unit 22 has a treatment instrument insertion port 223 communicating with the treatment instrument insertion passage formed inside the insertion unit 21 and used for inserting treatment instruments into the treatment instrument insertion passage.

Extending from the operating unit 22, the universal cable 23 includes a plurality of signal cables for transmitting various signals and an optical fiber for transmitting illumination light supplied from the light source apparatus 6.

The connector 24 is provided at the distal end of the universal cable 23. The connector 24 includes first to third connector units 241 to 243 each of which is connected with an ultrasound cable 31, a video cable 41, and an optical fiber cable 61, respectively.

The ultrasound observation apparatus 3 is electrically connected with the ultrasound endoscope 2 via the ultrasound cable 31, outputs a pulse signal to the ultrasound endoscope 2 via the ultrasound cable 31, while inputting echo signals from the ultrasound endoscope 2. Then, the ultrasound observation apparatus 3 performs predetermined processing on the echo signal and generates an ultrasound image.

The endoscopic examination apparatus 4 is electrically connected with the ultrasound endoscope 2 via the video cable 41, and inputs an image signal from the ultrasound endoscope 2 via the video cable 41. Then, the endoscopic examination apparatus 4 performs predetermined processing on the image signal and generates an endoscopic image.

The display device 5 is formed with liquid crystal, organic electroluminescence (EL), or the like, and displays an ultrasound image generated by the ultrasound observation apparatus 3, an endoscopic image generated by the endoscopic examination apparatus 4, or the like.

The light source apparatus 6 is connected with the ultrasound endoscope 2 via the optical fiber cable 61 and supplies illumination light for illuminating portions inside the subject, to the ultrasound endoscope 2 via the optical fiber cable 61.

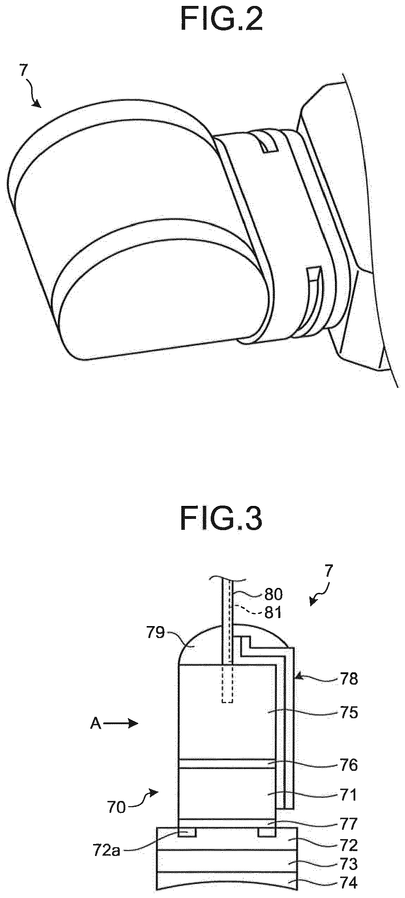

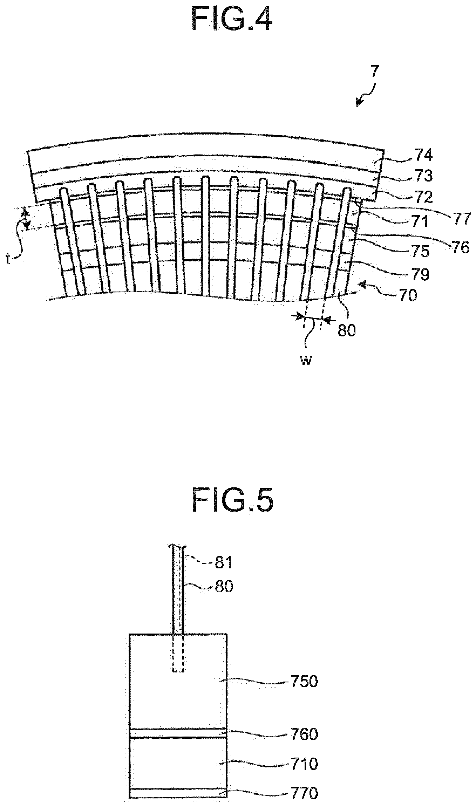

Subsequently, the configuration of the ultrasound transducer 7 will be described with reference to FIGS. 2 to 4. FIG. 2 is a perspective view illustrating the ultrasound transducer 7 according to the first embodiment. FIG. 3 is a schematic diagram illustrating a configuration of a main portion of the ultrasound transducer 7 according to the first embodiment. FIG. 4 is a schematic diagram illustrating a configuration of a main portion of the ultrasound transducer 7 according to the first embodiment, illustrated as a plan view in the direction of arrow A in FIG. 3, in an upside down view of FIG. 3. While FIG. 4 illustrates an exemplary case where ten elements 70 including the piezoelectric elements 71 are arranged for simplicity of explanation, the configuration of the ultrasound transducer 7 is not limited to this number in actual arrangement. The first embodiment is described as an exemplary case where the ultrasound transducer 7 is a convex ultrasound transducer as illustrated in FIG. 2 including a one-dimensional array (1D array) of the plurality of piezoelectric elements 71 which is arranged in a line. In other words, in the ultrasound transducer 7 according to the first embodiment, the plurality of elements 70 is arranged along an outer surface forming a curved surface of the ultrasound transducer 7.

As illustrated in FIGS. 3 and 4, the ultrasound transducer 7 includes the plurality of piezoelectric elements 71, a first acoustic matching layer 72 provided on an outer surface side of the ultrasound transducer 7 with respect to the piezoelectric elements 71, a second acoustic matching layer 73 provided on the side of the first acoustic matching layer 72 opposite to the side coming in contact with the piezoelectric element 71, an acoustic lens 74 provided on the side of the second acoustic matching layer 73 opposite to the side coming in contact with the first acoustic matching layer 72, a backing material 75 provided on the side of the piezoelectric element 71 opposite to the side coming in contact with the first acoustic matching layer 72, a first electrode 76 (signal input and output electrode) provided on a main surface of the piezoelectric element 71 on the backing material 75 side, a second electrode 77 provided on the main surface of the piezoelectric element 71 on the first acoustic matching layer 72 side, a conductive thin film 78 (connecting electrode) electrically connecting the first electrode 76 with a wiring pattern on an flexible printed circuit (FPC) substrate 80 to be described below, a sealing portion 79 for sealing a connecting portion between the conductive thin film 78 and the FPC substrate 80, and the FPC substrate 80 configured to perform input and output of electric signals on each of the piezoelectric elements 71. Note that the first embodiment has a configuration in which the first acoustic matching layer 72 and the backing material 75 are provided for each of the piezoelectric elements 71, and at the same time, the second acoustic matching layer 73 and the acoustic lens 74 integrally cover the plurality of piezoelectric elements 71 and the first acoustic matching layers 72. The element 70 in the first embodiment refers to an output unit including the piezoelectric element 71, the backing material 75, the first electrode 76, and the second electrode 77, and configured to output one ultrasound pulse according to a certain pulse signal. The first embodiment describes a case where one piezoelectric element 71 is an output unit. In a case, however, where ultrasound is simultaneously emitted from the plurality of piezoelectric elements 71 by a wiring pattern formed on the FPC substrate 80, a plurality of target piezoelectric elements 71 constitutes one element as an output unit.

The piezoelectric element 71 converts an electrical pulse signal into an ultrasound pulse (acoustic pulse), emits the ultrasound pulse to the subject, converts an ultrasound echo reflected on the subject into an electrical echo signal represented by a voltage change, and outputs the echo signal.

The piezoelectric element 71 is electrically connected with the FPC substrate 80 via the first electrode 76 by the conductive thin film 78. Each of the first electrode 76 and the second electrode 77 is formed of a metal material or a resin material, having conductivity.

The piezoelectric element 71 is formed with a PMN-PT single crystal, PMN-PZT single crystal, PZN-PT single crystal, PIN-PZN-PT single crystal, or a relaxer-based material. The PMN-PT single crystal is an abbreviation of a solid solution of lead magnesium niobate and lead titanate. The PMN-PZT single crystal is an abbreviation of a solid solution of lead magnesium niobate and lead zirconate titanate. The PZN-PT single crystal is an abbreviation of a solid solution of lead zinc-niobate and lead titanate. The PIN-PZN-PT single crystal is an abbreviation of a solid solution of lead indium niobate, lead zinc-niobate, and lead titanate. The relaxer-based material is a general term of a three-component piezoelectric material obtained by adding lead-based complex perovskite as a relaxer material to the lead zirconate titanate (PZT) for the purpose of increasing the piezoelectric constant and dielectric constant. The lead-based complex perovskite is represented by Pb(B1, B2)O₃, in which B1 is any of magnesium, zinc, indium, and scandium, while B2 is any of niobium, tantalum, and tungsten. These materials have excellent piezoelectric effects. These materials could reduce the value of the electrical impedance even in a miniaturized form, and thus, would be preferable from the viewpoint of impedance matching with a thin film electrode.

In order to allow the sound (ultrasound) to be efficiently transmitted between the piezoelectric element 71 and the observation target, the first acoustic matching layer 72 and the second acoustic matching layer 73 perform matching of acoustic impedance between the piezoelectric element 71 and the observation target. The first acoustic matching layer 72 and the second acoustic matching layer 73 are formed of mutually different materials. Note that while the first embodiment describes a case where there are two acoustic matching layers (first acoustic matching layer 72 and second acoustic matching layer 73), it is also allowable to have one layer or three layers or more, in accordance with characteristics of the piezoelectric element 71 and the observation target. Moreover, as for the acoustic matching layer, it is allowable to configure as an ultrasound transducer that does not include the acoustic matching layer as long as the acoustic impedance matching with the observation target can be achieved.

The acoustic lens 74 is formed with polymethylpentene, epoxy resin, polyetherimide, or the like, and has a function of narrowing the ultrasound with one side having a concave shape. Note that the material may be a material whose sound speed is slower than the sound speed of the subject, such as a silicone resin, with a convex surface that allows the ultrasound beam to converge. Whether to provide the acoustic lens 74 may be optional, and thus, it is allowable to have a configuration without the acoustic lens 74.

The backing material 75 attenuates unneeded ultrasound vibration generated by operation of the piezoelectric element 71. The backing material 75 is formed of a material having a high attenuation rate, for example, epoxy resin in which a filler such as alumina and zirconia is dispersed, or formed of a rubber in which the above-described filler is dispersed.

The first electrode 76 is electrically connected with the FPC substrate 80 via the above-described conductive thin film 78. The first electrode 76 is an electrode for inputting and outputting a signal with respect to the piezoelectric element 71.

The second electrode 77 is formed in the first acoustic matching layer 72 and is electrically connected to a ground electrode 72a grounded to the ground potential.

The conductive thin film 78 forms an electrical conduction path between the first electrode 76 and the FPC substrate 80. The conductive thin film 78 is a conductive thin film formed on a side surface of the piezoelectric element 71 by a physical vapor deposition (PVD) method such as sputtering and wet plating such as electrolytic plating, and configured to electrically connect the first electrode 76 with the wiring pattern formed on the FPC substrate 80. The conductive thin film 78 is obtained by forming a plating film on a stacked film formed of any of chromium/copper, chromium/gold, nickel-chromium/copper and chromium/copper/nickel.

The sealing portion 79 is formed with an insulating resin material and seals an outer surface of a portion of the backing material 75, and an outer surface of a portion of the FPC substrate 80 and the conductive thin film 78 including the connecting portion between the FPC substrate 80 and the conductive thin film 78.

The FPC substrate 80 is a substrate obtained by forming a wiring pattern formed of a conductive metal such as a copper foil on an insulating and flexible film-like base material formed of polyimide, or the like.

The piezoelectric element 71 vibrates with the input of the pulse signal, whereby the above-configured ultrasound transducer 7 emits ultrasound to the observation target via the first acoustic matching layer 72, the second acoustic matching layer 73, and the acoustic lens 74. At this time, the piezoelectric element 71 is configured such that the backing material 75 attenuates vibration of the piezoelectric element 71 on the side opposite to the arrangement side of the first acoustic matching layer 72, the second acoustic matching layer 73, and the acoustic lens 74, thereby suppressing transmission of vibration of the piezoelectric element 71 to the FPC substrate 80, or the like. Moreover, the ultrasound reflected from the observation target is transmitted to the piezoelectric element 71 via the first acoustic matching layer 72, the second acoustic matching layer 73, and the acoustic lens 74. The transmitted ultrasound causes the piezoelectric element 71 to vibrate, then, the piezoelectric element 71 converts the vibration into an electrical echo signal, and outputs the converted signal, as an echo signal, to the FPC substrate 80 via the conductive thin film 78.

Subsequently, a method of manufacturing the above-described ultrasound transducer 7 will be described with reference to FIGS. 5 to 12. FIGS. 5 to 10 are schematic diagrams illustrating a method of manufacturing the ultrasound transducer 7 according to the first embodiment. First, processing of producing a forming member (forming member 700 to be described below) for forming the piezoelectric element 71, the backing material 75, the first electrode 76, and the second electrode 77 will be described.

A first thin film 760 formed with a material forming the first electrode 76 and a second thin film 770 formed with a material forming the second electrode 77 are stacked on opposing main surfaces of a rectangular parallelepiped shaped piezoelectric element base material 710 formed with a material forming the piezoelectric element 71, and thereafter, a backing material base material 750 formed with a material forming the backing material 75 is stacked on the side of the first thin film 760 opposite to the piezoelectric element base material 710 side (refer to FIG. 5: stacked member production process). The backing material base material 750 is stacked while a portion of the FPC substrate 80 is embedded in the backing material base material 750.

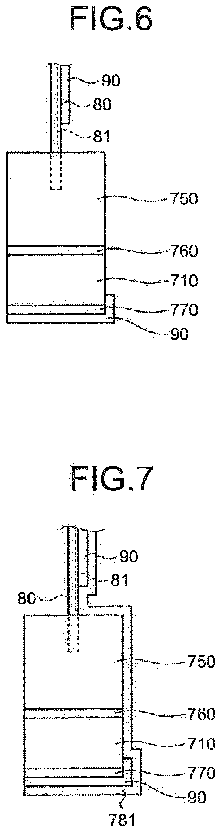

Thereafter, a masking material 90 to cover the second thin film 770 and a portion of the FPC substrate 80 is arranged (refer to FIG. 6). The masking material 90 may be anything as long as it masks the second thin film 770 and a portion of the FPC substrate 80 in a region where film forming is performed by sputtering to be described below. With this processing, film forming on the second thin film 770 by sputtering is prevented.

After the masking material 90 is arranged, a third thin film 781 is formed by sputtering using a material forming a portion of the conductive thin film 78 (refer to FIG. 7). Here, the third thin film 781 may include formation of gold (Au) with a thickness of 300 nm on an underlying layer of chromium (Cr) with a thickness of 50 nm, and include, on an underlying layer of nickel chromium (NiCr) with a thickness of 50 nm, stacks of copper (Cu) with a thickness of 100 nm and platinum (Pt) with a thickness of 400 nm, making it possible to form a conductive thin film with good adhesion. As a film forming method other than sputtering, silver (Ag) with a thickness of 1000 nm or silver palladium (AgPd) with a thickness of 700 nm may be formed by vapor deposition. By forming the third thin film 781, the first thin film 760 can be electrically connected with the wiring pattern formed on the FPC substrate 80.

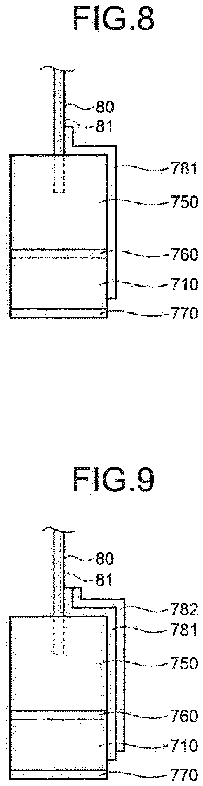

After the third thin film 781 is formed, the masking material 90 is removed (refer to FIG. 8), and then, a plating film 782 is formed by electrolytic plating treatment (refer to FIG. 9). A stacked film (connecting member) forming the conductive thin film 78 is formed by the third thin film 781 and the plating film 782 (connection electrode member arrangement process). Materials applicable as the plating film 782 is materials forming a portion of the conductive thin film 78 formed by a sulfamic acid bath or a pyrophosphoric acid bath, such as nickel and copper. With the electrolytic plating treatment, the third thin film 781 is covered with the plating film 782. In physical vapor deposition, it used to be difficult to increase the film thickness to lower the resistance due to an issue of film stress. More specifically, when the film stress is high, the film peels off during cutting by a precision cutting machine such as a dicing saw, making it difficult to form a thick film. In contrast, film stress can be controlled on the plating of nickel sulfamate or copper pyrophosphate, making it possible to form a thick film of 1 to 10 µm, and to ensure the thickness of the conductive film necessary as the wiring to the transducer. In other words, by using the plating film 782, it is possible to enhance physical characteristics such as the strength and the electrical characteristics of the conductive thin film 78. A stacked member is produced by the above-described processing, and a series of processing corresponds to the stacked member production process of the present invention.

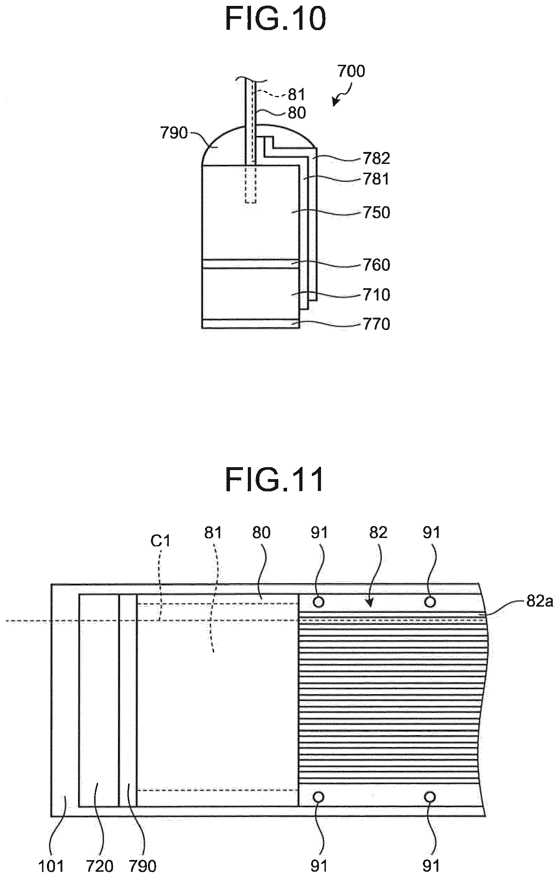

After formation of the plating film 782, a sealing member 790 is provided on a surface of the backing material base material 750, in which the FPC substrate 80 is embedded, so as to seal the outer surfaces of a portion of the FPC substrate 80 including the contact portion between the FPC substrate 80 and the third thin film 781, a portion of the third thin film 781, and a portion of the plating film 782, with the sealing member 790 (refer to FIG. 10: forming member production process). The forming member 700 is produced with the above-described processing.

FIG. 11 is a schematic diagram illustrating a method of manufacturing the ultrasound transducer 7 according to the first embodiment, viewed from the FPC substrate 80 side in FIG. 10. FIG. 11 is a top view illustrating a state in which a first acoustic matching layer base material 720 formed by stacking the second acoustic matching layer 73 is arranged on the second thin film 770 of the forming member 700 produced by the above-described processing, with the first acoustic matching layer base material 720 being mounted on a machining tool 101. The first acoustic matching layer base material 720 is formed of a material forming the first acoustic matching layer 72.

The FPC substrate 80 includes a foil-shaped solid portion 81 formed of a conductive material for forming wiring patterns and uniformly extending to a portion of the surface of the FPC substrate 80, and includes a pattern portion 82 in which a plurality of wiring lines 82a extend from the solid portion 81 in accordance with the individual wiring patterns. The solid portion 81 and the pattern portion 82 are formed of copper, for example. The above-described third thin film 781 is in contact with the solid portion 81.

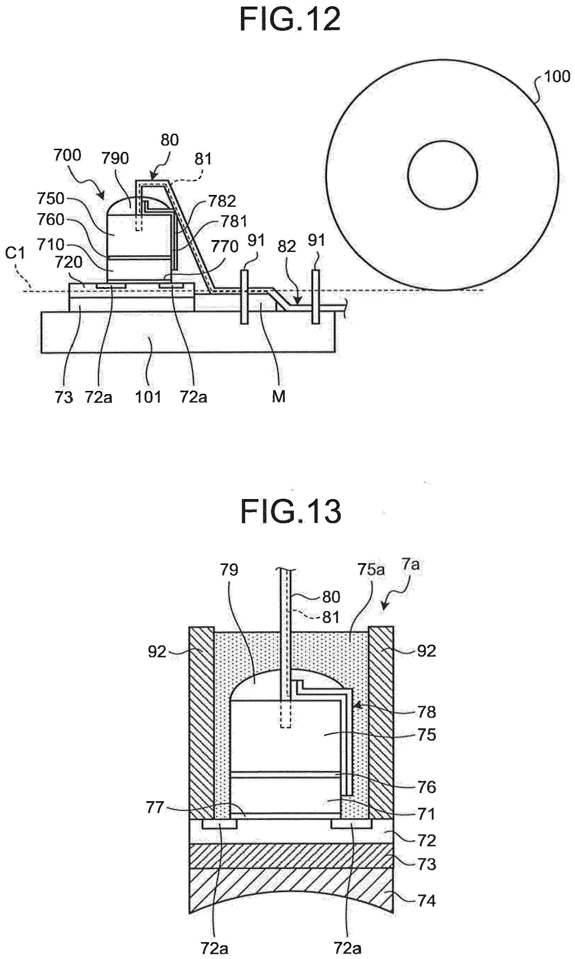

The FPC substrate 80 is positioned on the machining tool 101 by a positioning pin 91. At this time, the height of an end portion of the solid portion 81 connected to the pattern portion 82 is adjusted by a height adjusting member M (refer to FIG. 12) formed of machinable ceramics, or the like. Herein, the height is adjusted to the height at which a plane passing through the front surface of the FPC substrate 80, that is, the front surface coming in contact with the height adjusting member M, passes through the first acoustic matching layer base material 720.

FIG. 12 is a schematic diagram illustrating a method of manufacturing the ultrasound transducer 7 according to the first embodiment, as a side view from the arrangement direction of the individual wiring lines 82a of the pattern portion 82 in FIG. 11. As illustrated in FIG. 12, after arrangement of the FPC substrate 80, the forming member 700 connected to the FPC substrate 80, and the first acoustic matching layer base material 720 arranged on the forming member 700 and on which the second acoustic matching layer 73 is arranged, on the machining tool 101, cutting is performed on a portion of the FPC substrate 80 including the solid portion 81, the forming member 700, and the first acoustic matching layer base material 720 using a dicing saw 100. Specifically, as illustrated in FIGS. 11 and 12, by rotating and moving a blade of a precision cutting machine such as the dicing saw 100 along a cutting path C1 passing through the portions between the wiring lines 82a of the pattern portion 82 and extending in the longitudinal direction of the FPC substrate 80, a portion of the FPC substrate 80, the forming member 700, and the first acoustic matching layer base material 720 are cut and divided along a stacking direction of the forming member 700 (forming process). Note that the stacking direction herein refers to a stacking direction of the piezoelectric element base material 710, the first thin film 760, the second thin film 770, and the backing material base material 750. The solid portion 81 is divided by the dicing saw 100 in accordance with each of the wiring lines 82a and at the same time, the piezoelectric element 71, the first acoustic matching layer 72, the backing material 75, the first electrode 76, the second electrode 77, the conductive thin film 78 and the sealing portion 79 are formed, and thereafter, by arranging the acoustic lens 74, the ultrasound transducer 7 illustrated in FIGS. 3 and 4 is obtained.

The piezoelectric element 71 is formed by dividing the piezoelectric element base material 710 by the dicing saw 100. In this case, the piezoelectric element 71 has a rectangular parallelepiped shape, and when a length in the arrangement direction of the plurality of piezoelectric elements 71 in a plane orthogonal to the cut surface is w, and a length in the stacking direction of the first acoustic matching layer 72, or the like, orthogonal to the arrangement direction is t, it is preferable that a ratio represented by w/t is 0.3 to 0.7 in that this would achieve high electricity-machine conversion efficiency.

As described above, the first embodiment is a case of forming the forming member 700 including the piezoelectric element base material 710, the backing material base material 750, the first thin film 760, the second thin film 770, and the third thin film 781. The forming member 700 has a portion of the FPC substrate 80 including a contact portion between the FPC substrate 80 and the third thin film 781, a portion of the third thin film 781, and a portion of the plating film 782 being sealed with the sealing member 790, and thereafter, the forming member 700 is cut in accordance with the wiring line 82a together with the FPC substrate 80 including the solid portion 81. Since the piezoelectric element 71 and the FPC substrate 80 are electrically connected without using a bonding material that generates heat such as solder, it is possible to suppress degradation of the characteristics of the piezoelectric element 71 and to allow the space between the piezoelectric elements 71 to be about the thickness of the blade of the dicing saw 100, or the like. With this configuration, it is possible to realize a narrow pitch of the plurality of piezoelectric elements.

Moreover, according to the above-described first embodiment, the plurality of piezoelectric elements 71 and the FPC substrate 80 are connected with each other merely by cutting and dividing the solid portion 81 using the dicing saw 100. This configuration eliminates necessity of performing high-accuracy alignment of the piezoelectric element 71 and the wiring (for example, the wiring lines 82a), making it possible to perform production easily even when the pitch between the piezoelectric elements 71 is fine. This enables production of a high-quality ultrasound transducer for which narrow pitch is demanded.

In the first embodiment described above, it is also allowable to fix a relative relationship between the forming member 700 and the FPC substrate 80 by filling wax or the like between the forming member 700 and the FPC substrate 80.

While the above-described first embodiment is a case where the forming member 700 includes the piezoelectric element base material 710, the backing material base material 750, the first thin film 760, the second thin film 770, and the third thin film 781, the forming member 700 may further include the first acoustic matching layer base material 720, or the like.

### (First Modification of First Embodiment)

FIG. 13 is a schematic diagram illustrating a configuration of an ultrasound transducer according to a first modification of the first embodiment. While the above-described first embodiment is a case where the ultrasound transducer 7 includes one backing material (backing material 75), the ultrasound transducer 7 includes two backing materials in the first modification. As illustrated in FIG. 13, in the configuration of the ultrasound transducer 7 described above, a wall 92 having a hollow rectangular prism shape is built at the first acoustic matching layer 72, and a second backing material 75a is filled in a hollow space formed by the wall 92, thereby producing an ultrasound transducer 7a. The wall 92 encloses a plurality of oscillating portions including the piezoelectric element 71, the backing material 75, the first electrode 76, the second electrode 77, the conductive thin film 78, and the sealing portion 79. The second backing material 75a is provided in a hollow space formed by the wall 92 and the plurality of oscillating portions.

The acoustic impedance of the second backing material 75a is smaller than the acoustic impedance of the backing material 75 (first backing material). With application of the two backing materials having such a relationship, it is possible to cause the backing material 75 to hold the piezoelectric element 71 and to attenuate the unnecessary vibration with high efficiency, and to suppress transmission of vibration as a cause of crosstalk to the adjacent piezoelectric element 71 by the second backing material 75a. Therefore, according to the first modification, it is possible to realize reduction in the pulse width and suppression of crosstalk.

### (Second Modification of First Embodiment)

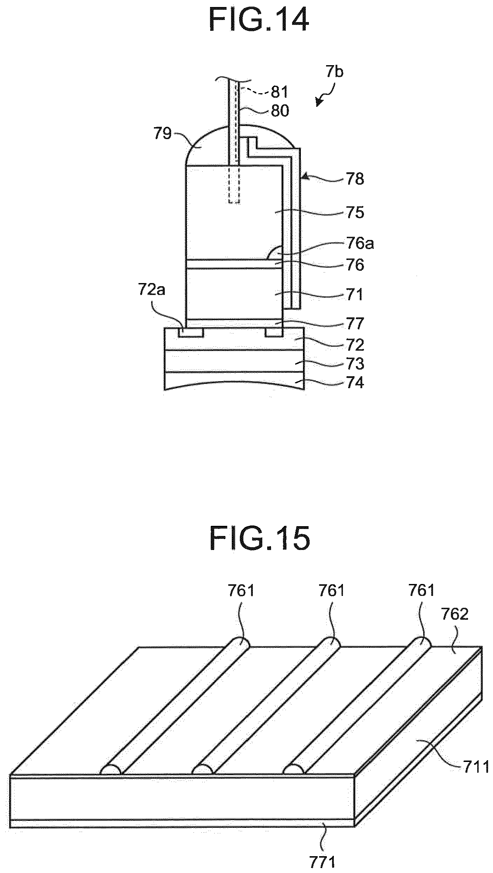

FIG. 14 is a schematic diagram illustrating a configuration of an ultrasound transducer according to a second modification of the first embodiment. While the above-described first embodiment is a case where the ultrasound transducer 7 includes the first electrode 76 having a flat plate shape, the second modification is a case where an ultrasound transducer 7b includes a thick portion 76a on the side of first electrode 76 coming in contact with the conductive thin film 78.

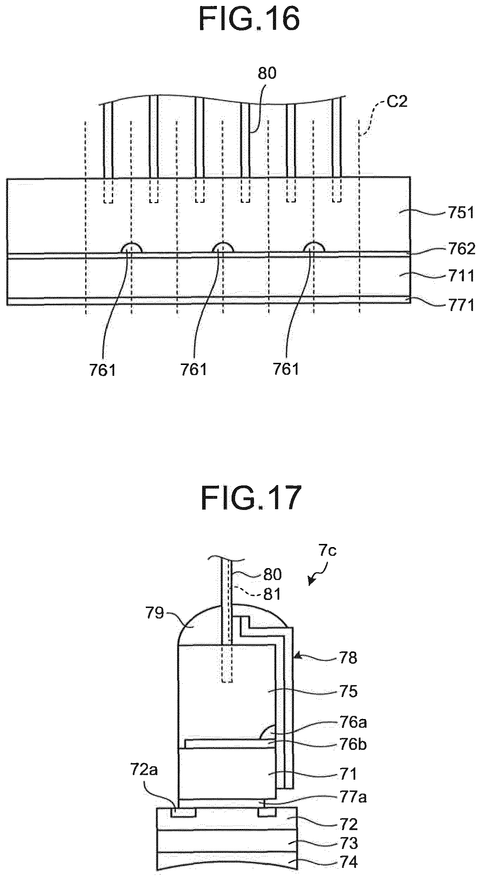

The thick portion 76a forms a portion of an electrical conduction path between the first electrode 76 and the FPC substrate 80. For example, the thick portion 76a is formed of the same conductive material as that of the first electrode 76, and comes in contact with the conductive thin film 78. FIGS. 15 and 16 are schematic diagrams illustrating production of a forming member of the ultrasound transducer according to the second modification of the first embodiment. As illustrated in FIG. 15, the forming member of the ultrasound transducer 7b according to the second modification is produced using a member having a structure in which a first thin film 762 having a plurality of protrusions 761 to be the thick portion 76a after the cutting processing is formed on one main surface of a piezoelectric element base material 711, while a second thin film 771 is formed on the other main surface of the piezoelectric element base material 711. Specifically, as illustrated in FIG. 16, after a backing material base material 751 is arranged on the first thin film 762, cutting is performed along a cutting path C2 passing through the protrusion 761, thereby obtaining a stacked body for forming the forming member including the piezoelectric element base material 710, the backing material base material 750, and the second thin film 770.

According to the second modification, by forming the thick portion 76a, the contact area between the first electrode 76 and the conductive thin film 78 is larger than the case of the first electrode 76 without the thick portion 76a described above, making it possible to provide further reliable electrical connection.

### (Third Modification of First Embodiment)

FIG. 17 is a schematic diagram illustrating a configuration of an ultrasound transducer according to a third modification of the first embodiment. While the above-described second modification is a case where the ultrasound transducer 7b includes the first electrode 76 having the thick portion 76a, the third modification is a case where an ultrasound transducer 7c includes a first electrode 76b and a second electrode 77a being exposed on each of opposing side surfaces.

The first electrode 76b has the thick portion 76a on an arrangement side of the conductive thin film 78 and is continuous to the side surface of the piezoelectric element 71 on the arrangement side of the conductive thin film 78 to be exposed to the outside, while retreating relative to the side surface of the piezoelectric element 71 on the side opposite to the side coming in contact with the conductive thin film 78. Moreover, the second electrode 77a is continuous to the side surface of the piezoelectric element 71 on the side opposite to the arrangement side of the conductive thin film 78 to be exposed to the outside, while retreating relative to the side surface of the piezoelectric element 71 on the arrangement side of the conductive thin film 78.

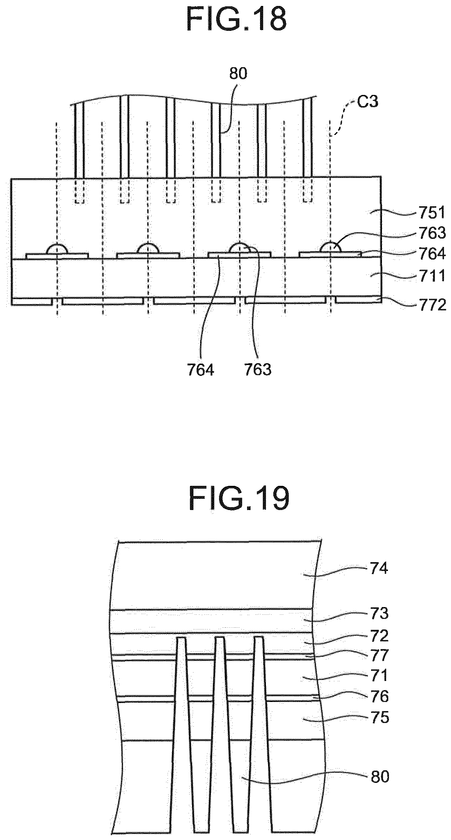

FIG. 18 is a schematic diagram illustrating production of a forming member of the ultrasound transducer according to the third modification of the first embodiment. As illustrated in FIG. 18, the forming member of the ultrasound transducer 7c according to the third modification is produced using a member having a structure in which a plurality of first thin films 764 having a protrusion 763 to be the thick portion 76a is formed on one main surface of the piezoelectric element base material 711, while a plurality of second thin films 772 is formed on the other main surface of the piezoelectric element base material 711. The first thin film 764 and the second thin film 772 are arranged in an alternating manner when viewed from the thickness direction of the piezoelectric element base material 711, with a space (gap) between the second thin films 772 being located at a position opposing the protrusion 763. The backing material base material 751 is arranged on the first thin film 764 having the protrusion 763, and thereafter, cutting is performed along a cutting path C3 passing through the protrusion 763, thereby obtaining a stacked body for forming the forming member.

### (Fourth Modification of First Embodiment)

FIG. 19 is a schematic diagram illustrating a configuration of a main portion of an ultrasound transducer according to a fourth modification of the first embodiment. While the above-described first embodiment is a case of the ultrasound transducer 7 in which a slit formed by cutting has a uniform width, the slit may be formed substantially in a V-shape having its width increasing toward the FPC substrate 80 side as in the fourth modification. According to the fourth modification, by widening a groove width on the FPC substrate 80 side, it is possible to facilitate casting operation of the second backing material after the cut stacked body is bent at the time of producing a convex transducer or a radial transducer.

### (Fifth Modification of First Embodiment)

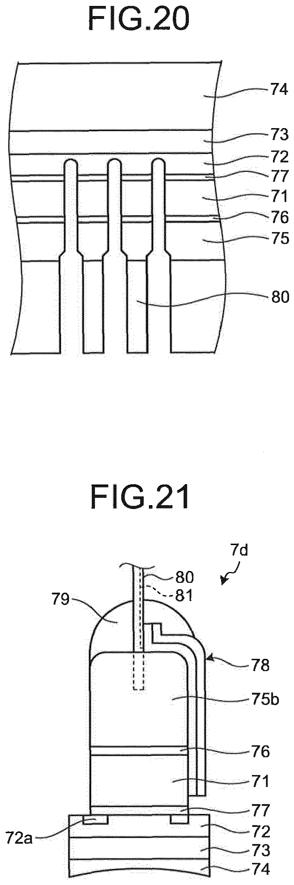

FIG. 20 is a schematic diagram illustrating a configuration of a main portion of an ultrasound transducer according to a fifth modification of the first embodiment. While the above-described first embodiment is a case of the ultrasound transducer 7 in which the slit formed by cutting has a uniform width, the slit may be formed in a stepped shape having its width increasing toward the FPC substrate 80 side as in the fifth modification.

### (Sixth Modification of First Embodiment)

FIG. 21 is a schematic diagram illustrating a configuration of an ultrasound transducer according to a sixth modification of the first embodiment, and is a diagram illustrating a configuration of a forming member. While the above-described first embodiment is a case where the backing material 75 has a prismatic shape, the backing material 75 may be a backing material 75b having a chamfered outer edge on the surface of the side holding the FPC substrate 80 as illustrated as an ultrasound transducer 7d according to the sixth modification. With this chamfering, it is possible to suppress damage on the conductive thin film 78.

### (Seventh Modification of First Embodiment)

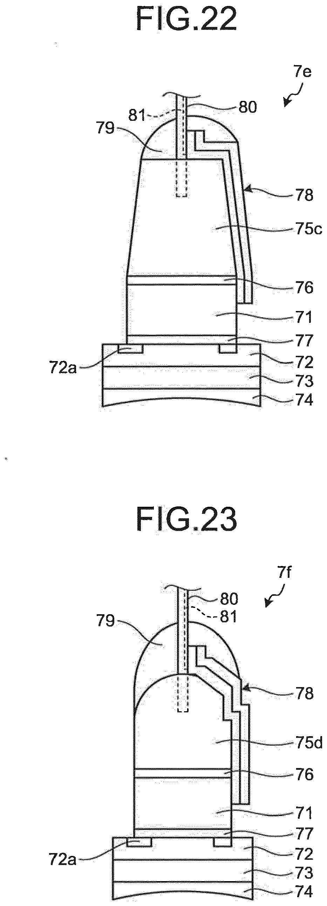

FIG. 22 is a schematic diagram illustrating a configuration of an ultrasound transducer according to a seventh modification of the first embodiment, and is a diagram illustrating a configuration of a forming member. While the above-described first embodiment is a case where the backing material 75 has a prismatic shape, the backing material 75 may be a backing material 75c having a trapezoidal shape in side view with the width decreasing toward the side holding the FPC substrate 80 as illustrated as an ultrasound transducer 7e according to the seventh modification.

### (Eighth Modification of First Embodiment)

FIG. 23 is a schematic diagram illustrating a configuration of an ultrasound transducer according to an eighth modification of the first embodiment and is a diagram illustrating a configuration of a forming member. While the above-described first embodiment is a case where the backing material 75 has a prismatic shape, the backing material 75 may be a backing material 75d having a curved surface on the side holding the FPC substrate 80 as illustrated as an ultrasound transducer 7f according to the seventh modification.

### (Ninth Modification of First Embodiment)

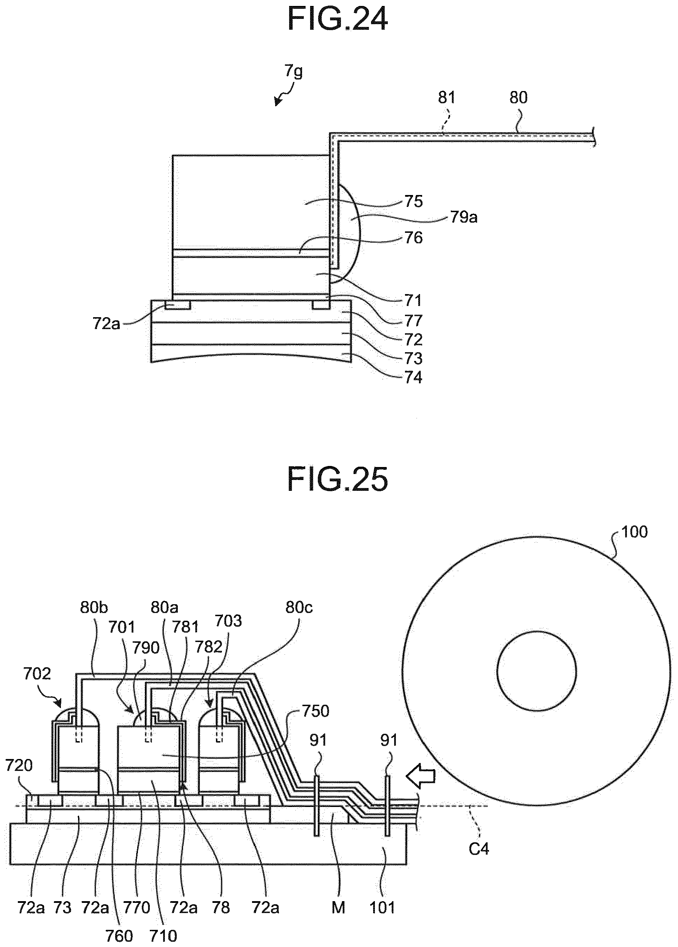

FIG. 24 is a schematic diagram illustrating a configuration of an ultrasound transducer according to a ninth modification of the first embodiment. While the above-described first embodiment is a case where the FPC substrate 80 is embedded in the backing material 75 and connected to the first electrode 76 via the conductive thin film 78, it is allowable to directly connect the first electrode 76 with the solid portion 81 after the division of the FPC substrate 80 by arranging the FPC substrate 80 on the side surface of the backing material 75 as in an ultrasound transducer 7g according to the ninth modification. In this case, a sealing portion 79a is formed at a connecting portion between the first electrode 76 and the FPC substrate 80.

Note that by applying the configuration of the ultrasound transducer 7b according to the above-described second modification also to the ninth modification, the contact region with the FPC substrate 80 is increased due to the thick portion 76a, making it possible to make the electrical connection further easy and stable.

### (Tenth Modification of First Embodiment)

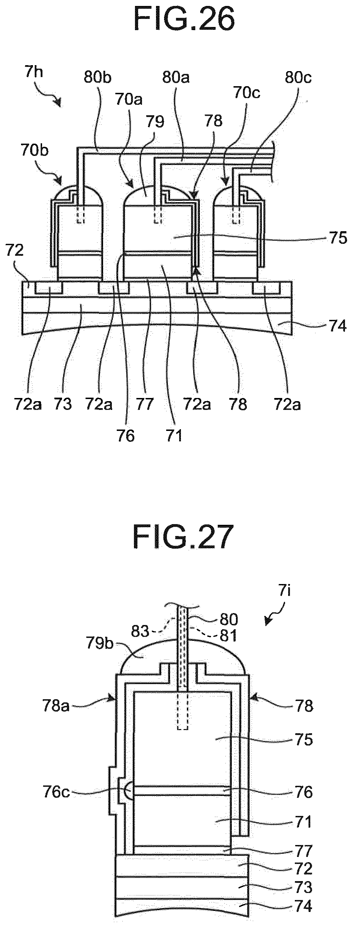

FIG. 25 is a diagram illustrating a method of manufacturing an ultrasound transducer according to a tenth modification of the first embodiment. FIG. 26 is a schematic diagram illustrating a configuration of the ultrasound transducer according to the tenth modification of the first embodiment. While the above-described first embodiment is an exemplary case of using a 1D array, application is also possible to a 1.25D array, a 1.5D array, or a 1.75D array in which a plurality of piezoelectric elements (oscillating portions) is arranged in a direction (elevation direction) substantially orthogonal to the scanning direction (arrangement direction of piezoelectric elements in the 1D array, the arrangement direction of piezoelectric elements along which the forming member 700 is divided) of the ultrasound transducer. Note that in a convex ultrasound transducer, the plurality of piezoelectric elements (oscillating portions) is arranged in a direction along a spherical surface in the arrangement direction of the piezoelectric elements, that is, a direction perpendicular to the scanning direction. For example, with a method of manufacturing a 1.25D-array ultrasound transducer illustrated in FIG. 25, there are provided three forming members as described above, for example, a first forming member 701, a second forming member 702, and a third forming member 703, respectively connected to mutually different FPC substrates 80a to 80c. The forming members 701 to 703 are arranged in the moving direction of the dicing saw 100 and thereafter individually divided along a cutting path C4 using the dicing saw 100, thereby achieving production of a 1.25D-array ultrasound transducer 7h having elements 70a to 70c as illustrated in FIG. 26.

Alternatively, the 1.25D-array ultrasound transducer 7h illustrated in FIG. 26 may be formed by building a wall serving as a weir at the time of casting of the backing material as illustrated in the first modification (refer to FIG. 13), and then, casting a portion between the groove and the wall divided by the dicing saw 100 using a liquid backing material and solidifying the backing material, so as to obtain a 1.25D-array ultrasound transducer. Examples of the liquid backing material include liquid materials obtained by mixing alumina (Al₂O₃), zirconia (ZrO₂), or the like, as a filler, with epoxy resin or silicone resin that can maintain flexibility after being cured. This also applies to ultrasound transducers with 1.5D array and 1.75D array in a similar manner.

### (Second Embodiment)

FIG. 27 is a schematic diagram illustrating a configuration of an ultrasound transducer according to a second embodiment. While the above-described first embodiment is a case where the second electrode 77 is connected to the ground electrode 72a provided in the first acoustic matching layer 72, the second embodiment is configured such that the second electrode 77 is electrically connected to a ground pattern 83 formed on the FPC substrate 80.

As illustrated in FIG. 27, an ultrasound transducer 7i according to the second embodiment includes a second conductive thin film 78a configured to connect the ground pattern 83 provided on a surface of the FPC substrate 80 opposite side of the connection surface of the conductive thin film 78 with the second electrode 77, in contrast with the above-described ultrasound transducer 7. Note that, in the second embodiment, the first acoustic matching layer 72 does not include the ground electrode 72a.

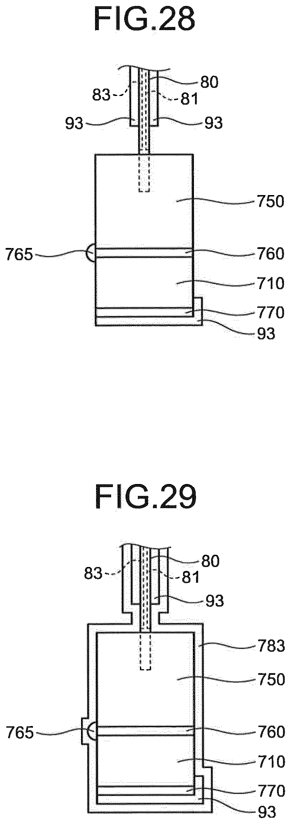

Next, a method of manufacturing the ultrasound transducer 7i will be described with reference to FIGS. 28 to 32. FIGS. 28 to 32 are schematic diagrams illustrating a method of manufacturing the ultrasound transducer according to the second embodiment. Herein, production of a forming member for forming the piezoelectric element 71, the backing material 75, the first electrode 76, the second electrode 77, the conductive thin film 78 (first conductive thin film) and the second conductive thin film 78a will be described.

First, as described above, the first thin film 760 and the second thin film 770 are formed on opposing main surfaces of the piezoelectric element base material 710, and thereafter, backing material base material 750 is provided on a side opposite to the piezoelectric element base material 710 side of the first thin film 760 (refer to FIG. 5).

Thereafter, a masking material 93 to cover the second thin film 770 and a portion of the FPC substrate 80 is arranged and a thin film formation prevention member 765 configured to prevent thin film formation by sputtering is arranged on a side of the first thin film 760, the side opposite to the formation surface of the conductive thin film 78 (refer to FIG. 28). A fourth thin film 783 is formed by sputtering using the material of the stacked film, being one constituent material of the conductive thin film 78 and the second conductive thin film 78a (refer to FIG. 29) .

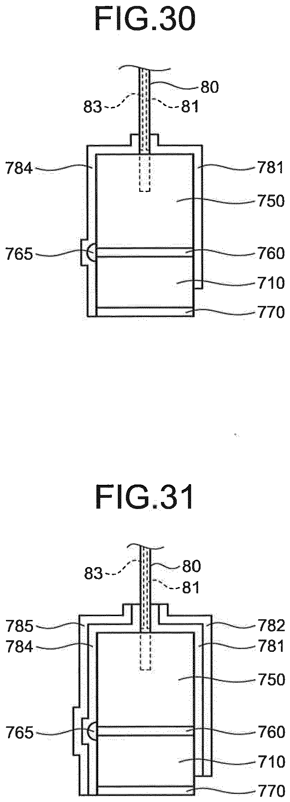

After formation of the fourth thin film 783, the masking material 93 is removed (refer to FIG. 30), leading to a state where the fourth thin film 783 is divided into the third thin film 781 connecting the first thin film 760 with the wiring pattern formed on the FPC substrate 80, and a fifth thin film 784 connecting the second thin film 770 with the ground pattern formed on the FPC substrate 80. Thereafter, the plating film 782 covering the third thin film 781 and a second plating film 785 covering the fifth thin film 784 are formed by electroplating treatment (refer to FIG. 31).

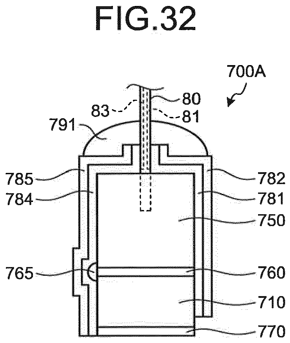

After formation of the plating film 782 and the second plating film 785, a sealing member 791 is provided on a surface of the backing material base material 750, on which the FPC substrate 80 is embedded, so as to seal a portion of the FPC substrate 80 including the contact portion between the FPC substrate 80 and each of the third thin film 781 and the fifth thin film 784, a portion of the third thin film 781 and a portion the fifth thin film 784, and a portion of the plating film 782 and a portion of the second plating film 785, by the sealing member 791 (refer to FIG. 32). A forming member 700A is produced by the above-described processing.

Thereafter, similarly to the above-described processing, the first acoustic matching layer base material 720 on which the second acoustic matching layer 73 is stacked is arranged on the second thin film 770 of the forming member 700A, so as to be mounted on the machining tool 101, and then, by rotating and moving the dicing saw 100 along the cutting path C1 (refer to FIGS. 11 and 12) extending in the longitudinal direction of the FPC substrate 80, a portion of the FPC substrate 80, the forming member 700A, and the first acoustic matching layer base material 720 are cut. The solid portion 81 and the ground pattern 83 are cut by the dicing saw 100 according to each of the wiring lines 82a and at the same time, the piezoelectric element 71, the first acoustic matching layer 72, the backing material 75, the first electrode 76, the second electrode 77, the conductive thin film 78, the second conductive thin film 78a, and a sealing portion 79b are formed, and thereafter, the acoustic lens 74 is arranged, thereby obtaining the ultrasound transducer 7i illustrated in FIG. 27. Note that a second sealing portion 76c formed by the thin film formation prevention member 765 is formed on the first electrode 76 on the second conductive thin film 78a side, thereby insulating between the first electrode 76 and the second conductive thin film 78a.

As described above, the second embodiment is a case of forming the forming member 700A including the piezoelectric element base material 710, the backing material base material 750, the first thin film 760, the second thin film 770, the fifth thin film 784, and the second plating film 785. The forming member 700A has a portion of the FPC substrate 80 including the contact portions between the FPC substrate 80 and each of the third thin film 781 and the fifth thin film 784, a portion of the first thin film 781 and a portion of the fifth thin film 784, and a portion of the plating film 782 and a portion of the second plating film 785 being sealed with the sealing member 791, and thereafter, the forming member 700A is cut in accordance with the wiring line 82a together with the FPC substrate 80 including the solid portion 81 and the ground pattern 83. Since the piezoelectric element 71 and the FPC substrate 80 are electrically connected without using a bonding material that generates heat such as solder, it is possible to suppress degradation of the characteristics of the piezoelectric element 71 and to allow the space between the piezoelectric elements 71 to be about the thickness of the blade of the dicing saw 100, or the like. With this configuration, it is possible to realize a narrow pitch of the plurality of piezoelectric elements.

Note that by applying the configuration of the ultrasound transducer 7c according to the third modification of the first embodiment described above also to the second embodiment, the backing material 75 (backing material base material 750) suppresses exposure of the side of the first electrode 76b opposite to the side coming in contact with the conductive thin film 78 to the outside. With this configuration, there is no need to provide the thin film formation prevention member 765, making it possible to produce an ultrasound transducer with the reduced number of components.

Embodiments of the present invention have been described hereinabove, however, the present invention is not intended to be limited to the above-described embodiments and the modification example. The present invention is not intended to be limited to the above-described embodiments and modification example but may include various forms of embodiments without deviating from the technical spirit and scope of the general inventive concept as defined in the appended claims of this invention. Furthermore, the components described in each of the embodiments and modification examples may be appropriately combined with each other.

In this manner, the present invention may include various forms of embodiments without deviating from the technical spirit and scope of the general inventive concept as defined in the appended claims of this invention.

### Industrial Applicability

As described above, the ultrasound transducer, the ultrasound probe, and the method of manufacturing the ultrasound transducer according to the present invention are useful for suppressing degradation of characteristics of the piezoelectric element and realizing narrow pitch of a plurality of the piezoelectric elements.

### Reference Signs List

- 1: ENDOSCOPE SYSTEM
- 2: ULTRASOUND ENDOSCOPE
- 3: ULTRASOUND OBSERVATION APPARATUS
- 4: ENDOSCOPIC EXAMINATION APPARATUS
- 5: DISPLAY DEVICE
- 6: LIGHT SOURCE APPARATUS
- 7 to 7i: ULTRASOUND TRANSDUCER
- 21: INSERTION UNIT
- 22: OPERATING UNIT
- 23: UNIVERSAL CABLE
- 24: CONNECTOR
- 31: ULTRASOUND CABLE
- 41: VIDEO CABLE
- 61: OPTICAL FIBER CABLE
- 70: ELEMENT
- 71: PIEZOELECTRIC ELEMENT
- 72: FIRST ACOUSTIC MATCHING LAYER
- 73: SECOND ACOUSTIC MATCHING LAYER
- 74: ACOUSTIC LENS
- 75: BACKING MATERIAL
- 75a: SECOND BACKING MATERIAL
- 76,: 76b FIRST ELECTRODE
- 76a: THICK PORTION
- 77,: 77a SECOND ELECTRODE
- 78: CONDUCTIVE THIN FILM
- 78a: SECOND CONDUCTIVE THIN FILM
- 79: SEALING PORTION
- 80, 80a to 80c: FPC SUBSTRATE
- 92: WALL
- 101: MACHINING TOOL
- 100: DICING SAW
- 211: RIGID MEMBER
- 212: BENDING PORTION
- 213: FLEXIBLE TUBE PORTION
- 221: BENDING KNOB
- 222: OPERATING MEMBER
- 223: TREATMENT INSTRUMENT INSERTION PORT
- 241: FIRST CONNECTOR UNIT
- 242: SECOND CONNECTOR UNIT
- 243: THIRD CONNECTOR UNIT
- 700, 700A: FORMING MEMBER
- 710, 711: PIEZOELECTRIC ELEMENT BASE MATERIAL
- 720: FOR FIRST ACOUSTIC MATCHING LAYER BASE MATERIAL
- 750, 751: FOR BACKING MATERIAL BASE MATERIAL
- 760, 762, 764: FIRST THIN FILM
- 761, 763: PROTRUSION
- 770, 771: SECOND THIN FILM
- 781: THIRD THIN FILM
- 782: PLATING FILM
- 783: FOURTH THIN FILM
- 784: FIFTH THIN FILM
- 785: SECOND PLATING FILM
- 790: SEALING MEMBER

## Claims

1. An ultrasound transducer comprising:
a plurality of piezoelectric elements configured to emit ultrasound according to an input of an electric signal and to convert ultrasound incident from outside into an electric signal;
a substrate configured to perform input and output of an electric signal with respect to each of the piezoelectric elements;
a plurality of signal input and output electrodes where each signal input and output electrode is provided between each piezoelectric element and the substrate and is configured to electrically connect the piezoelectric element with the substrate;
a plurality of backing materials where each backing material is provided at each of the piezoelectric elements on a side where the signal input and output electrode is arranged and is configured to attenuate ultrasound vibration generated by operation of the piezoelectric element; and
a plurality of sealing portions where each sealing portion is configured to seal an outer surface of at least a portion of an electrical path connecting the substrate with the signal input and output electrode,
wherein the plurality of piezoelectric elements, the plurality of backing materials, a portion of the substrate, the plurality of signal input and output electrodes, and the plurality of sealing portions are obtained by dividing a forming member along a stacking direction of the forming member, the forming member being formed by stacking a plurality of materials, each of the plurality of materials forming the piezoelectric elements, the backing materials, the substrate, the signal input and output electrodes, and the sealing portions.

2. The ultrasound transducer according to claim 1, further comprising:
a wall configured to surround a plurality of oscillating portions including at least the piezoelectric element, the backing material, the signal input and output electrode, and the sealing portion; and
a second backing material provided in a hollow space formed by the wall and the plurality of oscillating portions, and configured to attenuate the ultrasound vibration.

3. The ultrasound transducer according to claim 1 or 2,
wherein the signal input and output electrode includes a thick portion forming a portion of the electrical path.

4. The ultrasound transducer according to any one of claims 1 to 3, comprising a connection electrode connected to each of the substrate and the signal input and output electrode, and configured to form the electrical path.

5. The ultrasound transducer according to claim 4,
wherein a portion of the substrate is held by the backing material, and
the connection electrode is connected to the substrate via a side surface of the backing material.

6. The ultrasound transducer according to claim 4 or 5,
wherein the connection electrode includes a thin film formed by a physical vapor deposition method and a plating film formed by wet plating.

7. The ultrasound transducer according to claim 5,
wherein a portion of the substrate is embedded in the backing material.

8. The ultrasound transducer according to claim 5,
wherein a portion of the substrate is provided along a side surface of the backing material.

9. The ultrasound transducer according to claim 1, further comprising:
a second electrode paired with the signal input and output electrode; and
an acoustic matching layer provided at the piezoelectric element on a side opposite to a side on which the backing material is arranged and configured to adjust acoustic impedance of the ultrasound,
wherein the second electrode is grounded to a ground potential via conductive resin provided between the acoustic matching layer and the piezoelectric element.

10. The ultrasound transducer according to claim 1,
wherein the plurality of piezoelectric elements is arranged in a scanning direction along which the forming member is divided and in an elevation direction substantially orthogonal to the scanning direction.

11. An ultrasound probe comprising the ultrasound transducer according to any one of claims 1 to 10 at a distal end of the ultrasound probe.

12. A method of manufacturing an ultrasound transducer comprising:
a stacked member production process of stacking a plurality of materials to produce a stacked member, each of the materials forming:
a plurality of piezoelectric elements configured to emit ultrasound according to an input of an electric signal and to convert ultrasound incident from outside into an electric signal;
a portion of a substrate configured to perform input and output of an electric signal with respect to each of the piezoelectric elements;
a plurality of signal input and output electrodes where each signal input and output electrode is provided between each piezoelectric element and the substrate and is configured to electrically connect the piezoelectric element with the substrate; and
a plurality of backing materials where each backing material is provided at each of the piezoelectric elements on a side where the signal input and output electrode is arranged and is configured to attenuate ultrasound vibration generated by operation of the piezoelectric element;
a forming member production process of sealing, with respect to the stacked member, an outer surface of at least a portion of an electrical path connecting the substrate with the signal input and output electrode to produce a forming member; and
a forming process of dividing the forming member produced by the forming member production process along a stacking direction of the forming member to form the piezoelectric element, the backing material, a portion of the substrate, the signal input and output electrode, and the sealing portion.

13. The method of manufacturing an ultrasound transducer according to claim 12,
wherein the stacked member production process includes a connection electrode member arrangement process of arranging a connection electrode member for forming a connection electrode connected to each of the substrate and the signal input and output electrode, the connection electrode being configured to form the electrical path.

14. The method of manufacturing an ultrasound transducer according to claim 13,
wherein the connection electrode member arrangement process includes:
a first process of forming a thin film on an outer surface corresponding to the electrical path by a physical vapor deposition method; and
a second process of forming a plating film on an outer surface of the thin film by wet plating.

15. The method of manufacturing an ultrasound transducer according to claim 12,
wherein the forming member is obtained by stacking a material forming the piezoelectric element, a material forming the signal input and output electrode, and a material forming the backing material in this order, and by causing the substrate to be held by the backing material, and
the forming process includes cutting the forming member such that a distance between adjacent members formed by division increases in a direction from the piezoelectric element toward the substrate.
